# FASCICULE DE BREVET EUROPEEN

(11) **EP 3 595 546 B1**
(45) Date de publication et mention de la délivrance du brevet: **12.05.2021**
(21) Numéro de dépôt: 18726516.0
(22) Date de dépôt: 15.03.2018
(51) Int. Cl.: A61B 17/16, A61B 17/17

(54) **BROCHE DE GUIDAGE INTRA-OSSEUX, TROCART DE POSE ET TROCART DE RETRAIT DE LADITE BROCHE**
ENOSSALER FÜHRUNGSSTIFT, POSITIONIERUNGSTROKAR UND TROKAR ZUM ENTFERNEN DER STIFTES
ENDOSTEAL GUIDE PIN, POSITIONING TROCAR AND TROCAR FOR REMOVING SAID PIN

(30) Priorité: 15.03.2017 WO PCT/FR2017/050595
(43) Date de publication de la demande: 22.01.2020
(73) Titulaire: Hirondelle Medical, 13400 Aubagne (FR)
(72) Inventeur: DECOUX, Eric, 34980 Saint-Gély-du-Fesc (FR); HOA, Nguyen-Thanh Denis, 34980 Saint-Gély-du-Fesc (FR)
(74) Mandataire: Icosa
(86) Numéro de dépôt international: PCT/FR2018/050624
(87) Numéro de publication internationale: WO 2018/167435

(56) Documents cités:
- WO-A1-2016/114769
- US-A1- 2004 092 936
- US-A1- 2010 280 558
- US-A1- 2016 242 792

## Description

### Domaine de l'invention

L'invention s'inscrit dans le domaine de la chirurgie orthopédique et plus particulièrement dans le domaine de l'implantation de vis pédiculaires ou orthopédiques.

### Art antérieur

L'implantation de vis pédiculaires dans des vertèbres est largement utilisée en chirurgie orthopédique, notamment pour maintenir des vertèbres en place et/ou pour stabiliser des lésions de type fracture. On appelle également cette technique l'ostéosynthèse vertébrale par vis pédiculaire.

Les interventions chirurgicales dans ce domaine ont récemment évolué vers des techniques moins invasives qu'une exposition totale du champ opératoire. Ces nouvelles interventions dites mini-invasives évitent la perte de sang, les lésions des tissus, et réduisent le temps d'anesthésie et les risques infectieux. Si ces techniques mini-invasives nécessitent l'élaboration d'outils adaptés à une ouverture cutanée plus petite, il s'agit également de s'assurer du positionnement correct de la vis pédiculaire et de l'absence de lésion neurologique. Pour ce faire, l'intervention est couplée à un contrôle d'imagerie de type fluoroscopique, scanner ou IRM par exemple, accompagnant le guidage du positionnement précis de la vis pédiculaire.

La publication de brevet WO2009091616 décrit un outil chirurgical permettant la mise en place d'une broche de guidage provisoire servant de guide pour l'implantation des vis pédiculaires définitives. L'intervention peut être réalisée sur un patient en position couchée sur une table d'imagerie, et sous guidage radiologique par exemple par rayons X. L'outil comporte principalement un trocart de pose à l'extrémité duquel une broche de guidage provisoire est amoviblement et coaxialement montée. Une tige de fixation est également montée à l'intérieur du trocart de pose et de la broche de guidage. Cette tige de fixation comporte une pointe acérée qui, en faisant saillie de la broche de guidage, permet l'introduction de ladite broche. Un manchon muni d'une poignée est également prévu autour de la tige de fixation et du trocart de pose. Cet outil permet d'opérer en deux temps.

D'abord, la broche de guidage est introduite dans la vertèbre au moyen de l'outil et plus particulièrement du caractère amovible de la broche avec le reste de l'outil qui permet de larguer la broche de guidage depuis l'extrémité opposée de l'outil. La broche reste donc seule dans la vertèbre et peut, au moment de la pose des vis, être retirée. La vis pédiculaire est alors fixée dans la vertèbre au niveau de l'orifice préalablement préparé par la broche de guidage provisoire.

WO2016/114769 et US2016/242792 divulguent un trocart de pose d'une broche d'un de guidage intra-osseux.

### Inconvénients de l'art antérieur

L'instrument décrit dans cette publication présente un certain nombre d'inconvénients. En premier lieu, cet instrument est complexe de par le nombre de pièces impliquées, ce qui peut engendrer des difficultés pour assurer le maintien de la broche de guidage dans la vertèbre visée et qui induit des risques infectieux et des temps de procédures trop longs.

En outre, le nombre important de manipulations issues du nombre de pièces impliquées dans cet outil nécessite un espace d'intervention suffisamment large. Or la configuration de cet instrument nécessite que l'intervention, dans son ensemble, soit réalisée sous contrôle d'imagerie pour assurer l'insertion de la broche de guidage, son retrait, ainsi que l'insertion et la fixation de la vis pédiculaire. Il s'ensuit un encombrement important dans un espace de travail, par exemple un scanner, qui est limité.

Enfin, la complexité de l'outil en augmente le prix de fabrication.

### Objectifs de l'invention

L'invention vise une instrumentation composée d'une broche de guidage provisoire et de pièces d'introduction et de retrait de cette broche qui permettent d'assurer la mise en place aisée de la broche de guidage, ainsi que la mise en place ultérieure de la vis pédiculaire, sans se heurter aux problèmes visés précédemment.

En particulier, l'invention vise une instrumentation qui apporte dans sa globalité une meilleure précision et une meilleure sécurité pour le patient, sans être soumise aux difficultés précitées liées aux problèmes d'encombrement.

L'invention vise également une instrumentation simple pour un coût de fabrication réduit.

L'invention vise enfin une instrumentation apte à s'adapter à une méthode d'implantation de vis pédiculaire ou orthopédique dans laquelle les opérations de mise en place de la broche de guidage et de mise en place de la vis pédiculaire peuvent être dissociées.

### Exposé de l'invention

L'invention vise l'utilisation d'une broche de guidage intra-osseux pour préparer une trajectoire de guidage en vue de l'implantation d'une vis pédiculaire ou orthopédique, laquelle broche est essentiellement caractérisée en ce qu'elle comporte une tête destinée à être implantée de façon provisoire dans un os, et une tige de guidage coaxiale et solidaire de la tête, terminée par une extrémité libre adaptée pour au moins affleurer au niveau du plan cutané du patient lorsque la broche est en position implantée dans l'os, laquelle broche comporte un premier moyen de couplage destiné à assurer au moins sa solidarisation et désolidarisation avec au moins une partie d'un trocart de pose, et un second moyen de couplage destiné à assurer au moins sa solidarisation avec un trocart de retrait.

La broche de guidage de l'invention peut également comporter les caractéristiques optionnelles suivantes considérées isolément ou selon toutes les combinaisons techniques possibles :
- la tige de guidage de ladite broche est apte à être sectionnée afin que son extrémité se situe sous et à proximité du plan cutané lorsque la broche est en position implantée dans l'os ;
- au moins une partie de la tête présente une section circulaire de diamètre moyen D1, et la tige de guidage présente une section circulaire de diamètre moyen D2, inférieur au diamètre D1 de la tête ;
- la broche de guidage est réalisée en acier inoxydable ou en titane, et le diamètre D2 de la tige de guidage est inférieur à 2 millimètres ;
- la tête présente une extrémité libre constituée d'une pointe en biseau ou double biseau ou diamant ;
- le premier moyen de couplage comprend un filetage réalisé sur la tige de guidage ;
- le filetage est réalisé à distance de la tête ;
- le premier moyen de couplage comprend en outre un élément de blocage en rotation ;
- l'élément de blocage en rotation est réalisé au niveau de la tête ;
- l'élément de blocage en rotation est à proximité de la tige ;
- l'élément de blocage présente en section une forme polygonale, par exemple hexagonale ;
- le second moyen de couplage est constitué d'un filetage réalisé sur la tête ;
- le filetage est réalisé au niveau de l'élément de blocage ;
- l'élément de blocage comporte une partie lisse et une partie filetée formant également second moyen de couplage ;
- les premier et second moyens de couplage sont confondus.

L'invention porte également sur un trocart de pose d'une broche de guidage intra-osseux, qui est essentiellement caractérisé en ce qu'il comporte une première partie comprenant la broche de guidage, ladite broche comportant une tête destinée à être implantée de façon provisoire dans un os, et une tige de guidage coaxiale et solidaire de la tête, terminée par une extrémité libre, et adaptée pour au moins affleurer au niveau du plan cutané du patient lorsque la broche est en position implantée dans l'os, le trocart de pose comprenant en outre une seconde partie creuse à laquelle la première partie est couplée de façon amovible au moyen d'un système de solidarisation comportant un premier moyen de couplage ménagé sur ladite broche et un moyen de couplage coopérant ménagé sur le trocart de pose, la tige de guidage de la broche de guidage étant emmanchée à coulissement à l'intérieur de la partie creuse, et en ce que la broche comporte un second moyen de couplage destiné à assurer au moins sa solidarisation avec un trocart de retrait.

Avantageusement, l'extrémité libre de la partie creuse comporte des moyens d'enfoncement de la tête de guidage dans l'os, notamment des moyens d'entrainement en rotation, et le cas échéant de préhension.

De préférence, le premier moyen de couplage du système de solidarisation amovible comporte un filetage réalisé à distance de la tête de la broche de guidage, et en ce que le moyen de couplage coopérant comporte un taraudage coïncidant avec ledit filetage.

Plus préférentiellement, le taraudage est réalisé dans un écrou disposé coaxialement à ladite partie creuse du trocart de pose, lequel écrou coopère avec le filetage pour que la rotation de l'écrou entraîne le vissage ou dévissage de la tige de guidage de la broche relativement au reste du trocart de pose pour permettre la solidarisation ou la désolidarisation de la broche de guidage à la seconde partie creuse du trocart de pose.

Préférentiellement, le taraudage est situé au niveau des moyens d'enfoncement du trocart de pose.

Avantageusement, le système de solidarisation amovible comporte en outre un élément de blocage à rotation ménagé sur la broche et un élément de blocage coopérant ménagé dans la partie creuse du trocart pour empêcher la rotation de la broche de guidage par rapport à la partie creuse du trocart de pose selon l'axe dudit trocart de pose.

Plus préférentiellement, l'élément de blocage à rotation est ménagé au niveau de la tête de la broche.

Avantageusement, l'élément de blocage à rotation est situé à proximité de la tige.

De préférence, l'élément de blocage présente en section une forme polygonale, par exemple hexagonale, et en ce que l'élément de blocage coopérant est formé par une clé ménagée dans la partie creuse du trocart et adaptée pour coopérer avec le premier élément de blocage.

L'invention porte également sur un trocart de retrait d'une broche de guidage intra-osseux pour préparer une trajectoire de guidage en vue de l'implantation d'une vis pédiculaire ou orthopédique, la broche comportant une tête destinée à être implantée de façon provisoire dans un os, et une tige de guidage coaxiale et solidaire de la tête, terminée par une extrémité libre adaptée pour au moins affleurer au niveau du plan cutané du patient lorsque la broche est en position implantée dans l'os, laquelle broche comporte un premier moyen de couplage destiné à assurer sa solidarisation et désolidarisation avec au moins une partie d'un trocart de pose, et un second moyen de couplage destiné à assurer au moins sa solidarisation avec un trocart de retrait, caractérisé en ce qu'il comporte une tige creuse d'attache destinée à s'emmancher autour de la tige de guidage de la dite broche, et un moyen de couplage coopérant avec le second moyen de couplage de la dite broche pour former un système de solidarisation amovible permettant le retrait de la broche de guidage lorsque le second moyen de couplage de la broche et le moyen de couplage coopérant de la tige creuse du trocart de retrait sont assemblés.

Avantageusement, le moyen de couplage coopérant est constitué d'un taraudage réalisé sur la face interne de la tige creuse, et qui est apte à être assemblé au second moyen de couplage de la broche de guidage constitué par un filetage réalisé sur la tige de guidage ou sur la partie d'extrémité de la tête solidaire de la tige de guidage.

L'invention concerne également sur un procédé de préparation d'une trajectoire de guidage en vue de l'implantation d'une vis pédiculaire ou orthopédique, comprenant les étapes suivantes de :
a. Insertion du trocart de pose décrit précédemment ;
b. Désolidarisation et retrait de la seconde partie creuse du trocart de pose de manière à ne laisser que la broche implantée dans l'os ;
c. Sectionnement de l'extrémité libre de la tige de la broche de sorte que la tige sectionnée se situe sous et à proximité du plan cutané

Préférentiellement, le procédé comprend une étape supplémentaire de mise en place du trocart de retrait décrit précédemment et solidarisation de ce dernier à la broche de guidage par le système de solidarisation amovible considéré, suivie d'une étape de retrait de la broche à l'aide du trocart de retrait.

Avantageusement, l'étape de désolidarisation de la partie creuse du trocart de pose est réalisée par dévissage de l'écrou du filetage de l'extrémité libre de la tige, suivi du retrait par translation le long de l'axe de la tige de ladite partie creuse.

De préférence, la mise en place du trocart de retrait est réalisée par vissage dudit trocart autour du filetage réalisé sur la tige ou sur la tête de la broche.

### Liste des figures

D'autres caractéristiques et avantages de l'invention ressortiront clairement de la description qui en est donnée ci-dessous, à titre indicatif et nullement limitatif, en référence aux figures annexées parmi lesquelles :
- la figure 1 est une représentation schématique de coté de la broche de guidage de l'invention selon une première variante,
- la figure 2 est une représentation schématique de côté d'un trocart de pose de broche de guidage de l'invention selon une première configuration intégrant en première partie ladite broche de la figure 1, et illustré lorsque la broche est solidaire au trocart de pose,
- la figure 3 est une représentation schématique de côté du trocart de pose de broche de guidage de l'invention de la figure 2, illustré lorsque la broche est désassemblée du trocart de pose,
- la figure 4 est une représentation schématique de côté du trocart de retrait de l'invention selon une première configuration, emmanché autour de la tige de guidage de la broche de l'invention de la figure 1 et selon une position intermédiaire de désassemblage

Les figures 5 à 12 illustrent les opérations d'implantation dans une vertèbre et de retrait de la broche de l'invention de la première variante illustrée sur la figure 1, mettant en jeu le trocart de pose selon la première configuration illustrée sur les figures 2 et 3, et le trocart de retrait selon la première configuration illustrée sur la figure 4.

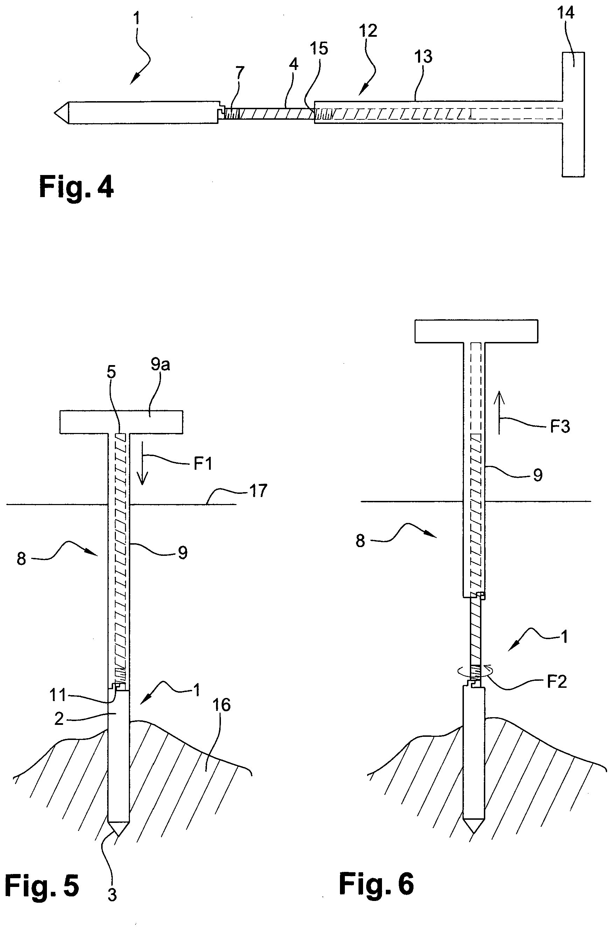

- la figure 5 est une représentation schématique d'une première étape de mise en place de la broche de guidage dans une vertèbre, dans laquelle étape la première partie du trocart de pose constituée par la tête de la broche de l'invention est implantée dans la vertèbre,
- la figure 6 une représentation schématique d'une deuxième étape de mise en place de la broche de guidage dans une vertèbre, dans laquelle étape la partie creuse du trocart de pose a été désassemblé de la broche de guidage et est extrait,

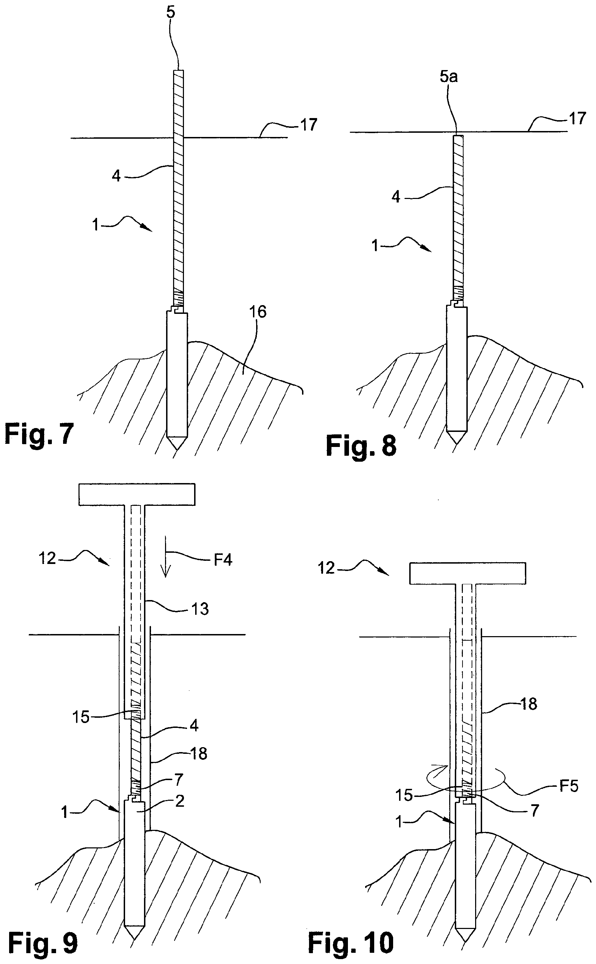

- la figure 7 est une représentation schématique de la broche de guidage en place dans la vertèbre, la tige de guidage de la broche dépassant du plan cutané,
- la figure 8 est une représentation schématique d'une troisième étape de mise en place de la broche de guidage dans une vertèbre, dans laquelle la tige de guidage de la broche est sectionnée sous et à proximité du plan cutané,
- la figure 9 est une représentation schématique d'une première étape de retrait de la broche impliquant la mise en place préalable d'une gaine externe et illustrant l'introduction d'un trocart de retrait selon l'invention autour de la tige de guidage de la broche en position,
- la figure 10 est une représentation schématique d'une deuxième étape de retrait de la broche, dans laquelle étape le trocart de retrait est solidarisé à la broche,

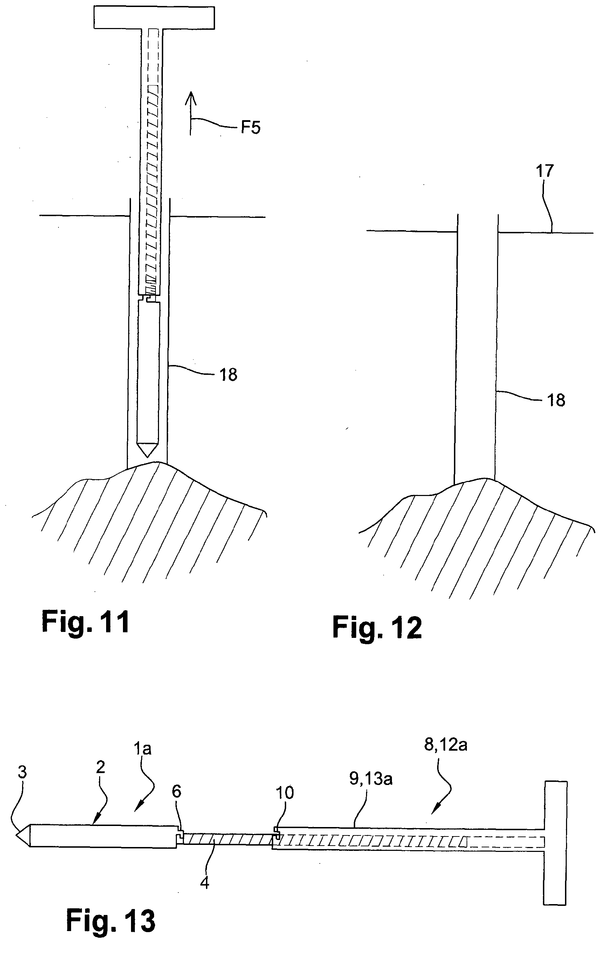

- la figure 11 est une représentation schématique d'une deuxième étape de retrait de la broche, dans laquelle étape le trocart de retrait et la broche sont extraits,
- la figure 12 est une représentation schématique de la gaine externe en position permettant de guider l'opérateur pour la mise en place d'une vis pédiculaire,

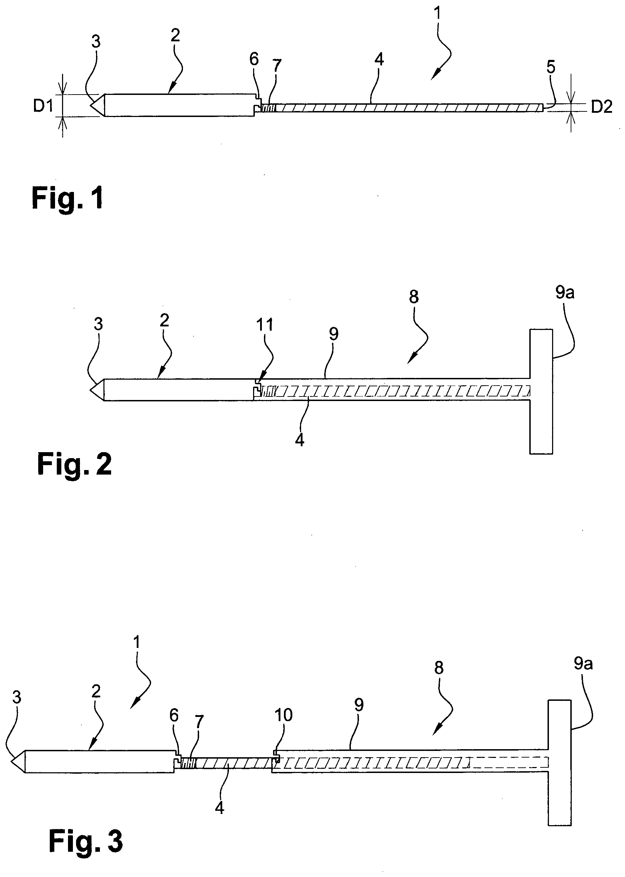

- la figure 13 est une représentation schématique identique à celle de la figure 3 illustrant un trocart de pose d'une broche de guidage selon une configuration avantageuse dans laquelle la partie creuse de ce trocart de pose forme trocart de retrait,

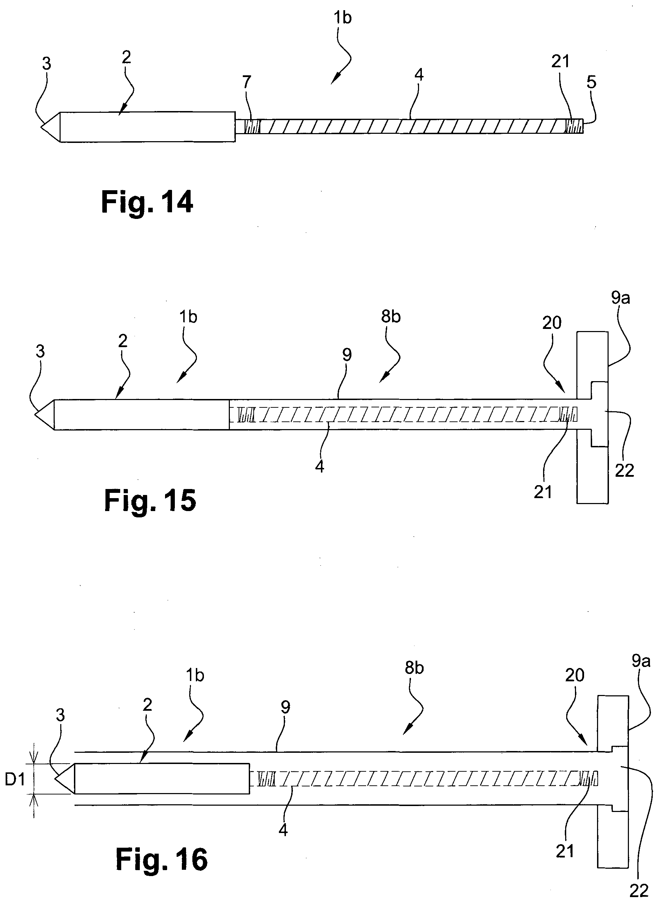

- la figure 14 est une représentation schématique de coté de la broche de guidage de l'invention selon une seconde variante,
- la figure 15 est une représentation schématique de côté d'un trocart de pose de broche de guidage de l'invention selon une deuxième configuration intégrant en première partie la broche de la figure 14, et illustré lorsque la broche est solidaire au trocart de pose,
- la figure 16 est une représentation schématique de côté d'un trocart de pose de broche de guidage de l'invention selon une troisième configuration intégrant en première partie la broche de la figure 14, et illustré lorsque la broche est solidaire au trocart de pose,

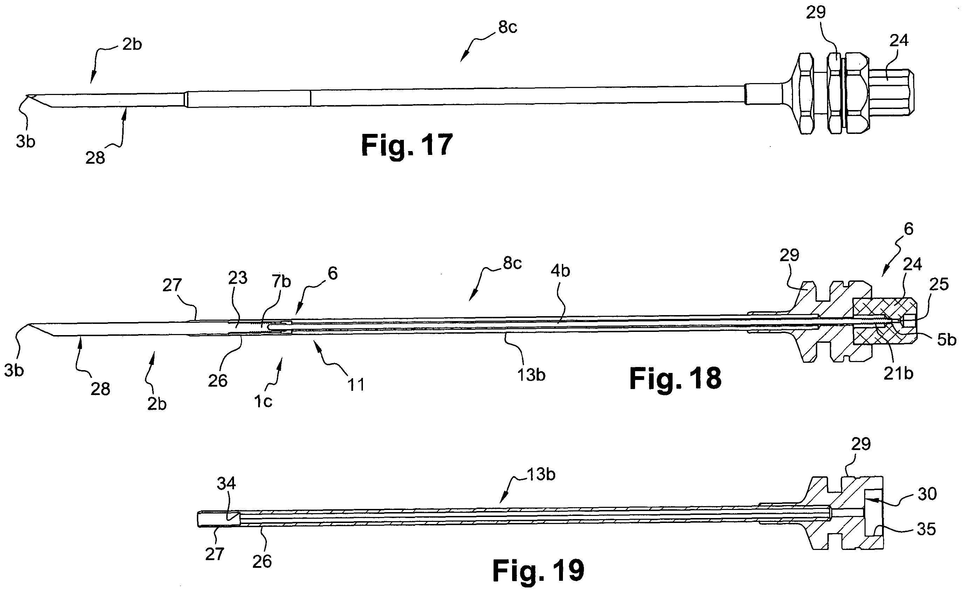

- la figure 17 est une représentation de côté d'un trocart de pose de broche de guidage de l'invention selon une quatrième configuration,
- la figure 18 est une représentation en coupe longitudinale du trocart de pose de la figure 17,
- la figure 19 est une représentation en coupe longitudinale d'un détail de la figure 17, illustrant la partie creuse du trocart de pose,

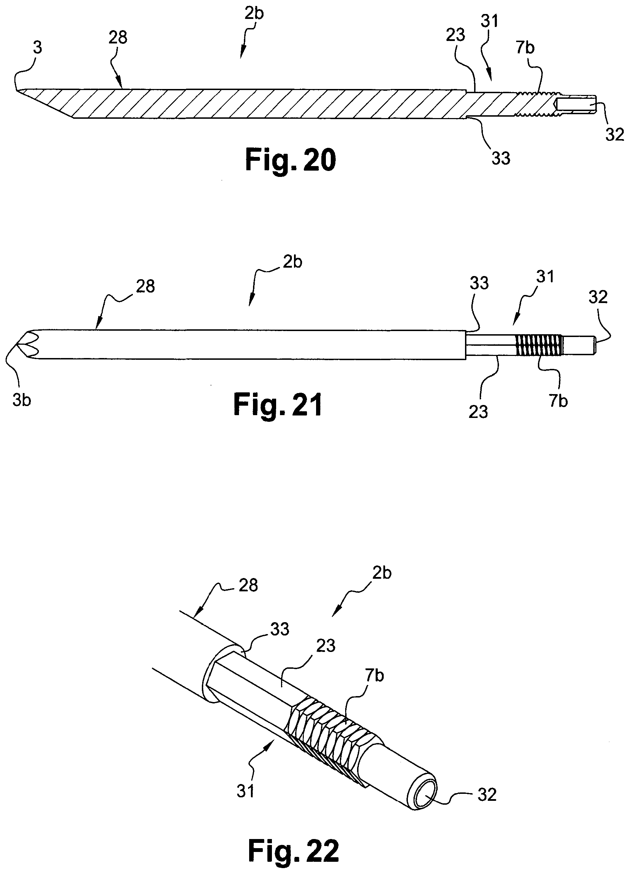

- la figure 20 est une représentation en couple longitudinale de la tête de la broche de guidage de la figure 17,
- la figure 21 est une vue de dessus de la tête de la broche de guidage de la figure 17, et
- la figure 22 est une vue en perspective d'une partie de la tête de la broche de guidage de la figure 17, montrant notamment la portion de jonction avec la tige de guidage, un élément de blocage hexagonal et le second moyen de couplage formé par un filetage hexagonal.

### Description détaillée de l'invention

L'instrumentation de l'invention prévoit en premier lieu une broche de guidage intra-osseux de configuration inédite qui présente une extension coaxiale de la tête de broche apte à s'étendre sous et à proximité du plan cutané du patient lorsque la tête est impactée dans l'os. Ainsi, l'opérateur peut aisément retrouver la broche pour la mise en place de la vis pédiculaire.

L'instrumentation de l'invention prévoit également un trocart de pose qui intègre ladite broche de guidage. Plus particulièrement, le trocart de pose comprend une première partie formée par la broche de guidage et une seconde partie comportant au moins une tige creuse. La première partie comprend une partie distale du trocart de pose constituée par la tête de la broche de guidage. L'extrémité de cette partie distale, et donc l'extrémité libre de la tête de la broche, peut comporter une pointe en simple biseau, ou de préférence en double biseau qui permet à la fois un passage cutané unique lors de l'introduction du trocart, et un impact optimisé de la broche dans l'os. Alternativement, l'extrémité libre de la tête de la broche est une pointe diamant, particulièrement adaptée à l'implantation de la tête de la broche avec un trocart de pose associé à un système d'enfoncement motorisé. L'intégration de la pointe à la broche permet de s'affranchir de l'utilisation d'une tige de fixation prévue dans le système de l'art antérieur précédemment décrit.

En outre, la broche et le reste du trocart sont solidarisés par des moyens de couplage qui peuvent être pour la variante illustrée sur les figures 1 à 12, un système de solidarisation à baïonnette. Ce système n'engendre pas les problèmes de tenue mécanique du pas de vis du porte broche de l'art antérieur, ce qui permet de motoriser l'introduction du trocart de pose et l'implantation de la broche dans l'os. On peut prévoir d'autres moyens de fixation, par exemple un système de filetage/taraudage et écrou situés à proximité de l'extrémité libre de la seconde partie ou partie creuse du trocart de pose (figures 14 à 16), avec le cas échéant des moyens de blocage empêchant tout mouvement de rotation de la broche de guidage selon son axe par rapport au reste du trocart de pose (figures 20 à 22).

Enfin, l'instrumentation de l'invention prévoit également un trocart de retrait de la broche. Selon une variante, ce trocart de retrait peut être constitué par la seconde partie du trocart de pose. Dans cette hypothèse, l'instrumentation complète comporte la seconde partie d'un trocart de pose formant également trocart de retrait, et la broche de guidage formant première partie du trocart de pose et dont la tête forme partie distale du trocart de pose. L'instrumentation est alors constituée seulement de deux pièces.

L'instrumentation de l'invention permet de dissocier les opérations de mise en place de la broche de guidage et de mise en place de la vis pédiculaire. La broche de guidage peut ainsi, par exemple, être mise en place sous contrôle d'imagerie par un premier opérateur, puis le patient transporté dans un autre lieu. Les opérations de retrait de la broche de guidage et de fixation de la vis pédiculaire peuvent être réalisées par un second opérateur, dans un autre lieu, par exemple en salle d'opération, sans contrôle d'imagerie. Il est ainsi possible de bénéficier, pour la pose de la broche de guidage, des appareils d'imagerie de guidage qui ne sont pas disponibles en salle d'opération. En outre, la pose de la vis pédiculaire n'est pas soumise à des contraintes d'encombrement.

En référence à la figure 1, la broche de guidage de l'invention 1 comporte une tête 2 de section circulaire présentant un diamètre D1 et terminée à son extrémité libre par une pointe en double biseau 3 ou une pointe diamant. La broche 1 se prolonge du côté opposé à la pointe 3 par une tige de guidage coaxiale 4 de section circulaire présentant un diamètre D2 inférieur au diamètre D1 de la tête 2 et se terminant par une extrémité libre 5. La broche de guidage 1 s'étend donc longitudinalement depuis la pointe 3 de la tête 2 jusqu'à l'extrémité libre 5 de la tige de guidage 4.

L'extrémité opposée à la pointe 3 de la tête 2 comporte un premier moyen de couplage constitué par la partie mâle 6 d'un système de solidarisation à baïonnette qui est destiné à coopérer avec la partie femelle de ce système ménagée sur la partie creuse du trocart de pose qui sera décrit plus loin.

La tige de guidage 4 comporte quant à elle un second moyen de couplage constitué par un filetage 7 destiné à coopérer avec un taraudage ménagé sur le trocart de retrait de l'invention qui sera décrit plus loin. De manière alternative, comme cela est représenté sur les figure 17 à 22, le filetage 7b destiné à coopérer avec le taraudage du trocart de retrait est réalisé sur une partie de la tête 2b de la broche de guidage située à proximité de la tige de guidage 4b.

La broche de guidage est réalisée en un matériau métallique, par exemple en acier inoxydable ou en titane. À titre d'exemple, la longueur totale de la broche est de 150 millimètres, le diamètre D1 de 2,4 millimètres et le diamètre D2 de 1 millimètre. La longueur de la tige de guidage 4 est quant à elle d'au moins 100 millimètres pour dépasser ou au moins affleurer le plan cutané lorsque la tête de la broche est en position implantée dans l'os (avant toute opération de coupe de la tige de guidage)

En référence aux figures 2 et 3, le trocart de pose de l'invention 8 comporte une seconde partie dite partie creuse constituée d'une tige creuse 9 et terminée par une poignée de préhension et d'enfoncement 9a. Cette poignée 9a permet d'introduire et de retirer manuellement le trocart de pose 8. On peut prévoir à la place de cette poignée 9a des moyens de coopération avec un outil motorisé pour assurer la mise en place et le retrait motorisé du trocart 8.. De tels moyens de coopération avec un outil motorisé sont représentés sur les figures 17 à 19, et sont formés par un organe d'entrainement en rotation 29 solidaire de l'extrémité libre de la partie creuse du trocart de pose 8c et destiné à coopérer avec l'embout formant clé de l'outil motorisé, par exemple une visseuse motorisée. Pour assurer la mise en prise de l'organe d'entrainement en rotation 29 avec l'embout de la visseuse, ledit organe 29 présente en section une forme polygonale, préférentiellement hexagonale.

À l'extrémité opposée de la poignée 9a, la partie creuse 9 se prolonge coaxialement par une première partie constituée par la broche 1 dont la tige de guidage 4 est emmanchée à coulissement dans la partie creuse 9 du trocart 8.

Par ailleurs et en référence à la figure 3, l'extrémité opposée à la poignée 9a de la partie creuse 9 du trocart 8 présente une partie femelle 10 d'un système de solidarisation à baïonnette qui coopère avec la partie mâle 6 située à l'extrémité de la tête 2 de la broche 1. La coopération de ces deux parties 6,10 forme (figure 2) le système de solidarisation à baïonnette 11 permettant de solidariser et désolidariser par rotation et retrait longitudinal la partie creuse 9 du trocart de pose 8 de la broche 1.

Ainsi, le trocart de pose 8 intègre la broche de guidage 1 de la figure 1 qui est, via le système de solidarisation à baïonnette 11, solidarisée ou désolidarisée de la partie creuse 9 du trocart 8.

En référence à la figure 4, le trocart de retrait de la broche 12 selon une première variante est fait d'une tige creuse 13 terminée à l'une de ses extrémités par une poignée de préhension 14. On peut prévoir à la place de cette poignée 14 des moyens de coopération avec un outil motorisé pour assurer la mise en place et le retrait motorisé du trocart 12, par exemple identiques à ceux décrits plus haut relativement à la partie creuse du trocart de pose.

À l'extrémité opposée de la poignée 14, le pourtour interne du tube creux 13 comporte un taraudage 15 qui est apte à coopérer avec le filetage 7 ménagé sur la tige de guidage 4 de la broche 1 et à proximité de sa tête 2. Le diamètre interne du tube creux 13 du trocart de retrait 12 est ainsi adapté à l'emmanchement du trocart de retrait 12 autour de la tige de guidage 4 de la broche 1 et à la coopération du taraudage 15 avec le filetage 7. Le diamètre externe du tube creux n'est lui, soumis à aucune contrainte de coopération avec la broche 1. Alternativement, le trocart de retrait et son taraudage sont adaptés pour coopérer avec le filetage 7b réalisé sur la tête 2b de la broche de guidage, à proximité de la tige de guidage 4b (figures 17 à 22).

On se réfère aux figures 5 à 12 pour décrire les différentes étapes de mise en place de la broche provisoire 1 dans l'os et de retrait de cette broche par un opérateur ou deux opérateurs avant l'implantation de la vis pédiculaire ou orthopédique.

Sur la figure 5, le trocart de pose 8 muni à son extrémité de la broche 1 est introduit facilement selon la flèche F1 grâce à la pointe en double biseau 3. La broche est enfoncée dans l'os 16 manuellement ou de façon motorisée, et la poignée 10 du trocart de pose 8 dépasse naturellement du plan cutané 17, ainsi que l'extrémité 5 de la tige de guidage 4 de la broche 1.

Sur la figure 6, le retrait de la partie creuse 9 du trocart de pose 8 est réalisé en tournant la poignée 9a du trocart de pose 8 dans le sens antihoraire selon la flèche F2 pour assurer la désolidarisation des parties mâle et femelle du système de solidarisation à baïonnette 11 et le largage de la broche 1 de la partie creuse 9 du trocart de pose 8, puis en réalisant un effort de traction selon la flèche F3.

Comme représenté sur la figure 7, la broche 1 reste alors en place dans l'os 16, l'extrémité 5 de la tige de guidage 4 dépassant du plan cutané 17.

On sectionne alors avec un outil approprié, par exemple une pince coupante, la zone d'extrémité de la tige de guidage 4 afin que la nouvelle extrémité 5a se situe sous et à proximité du plan cutané 17. Ce positionnement permet d'une part à l'opérateur, ou à un second opérateur de pouvoir retrouver cette extrémité par simple palpation, et d'autre part d'éviter tout risque infectieux si le patient est amené dans un second lieu pour la suite des opérations.

Toutes ces opérations décrites en référence aux figures 5 à 8, et plus particulièrement celles des figures 5 et 6 sont de préférence réalisées sous guidage par imagerie, scanner, radioscopique ou IRM, par un radiologue. La position de la broche 1 dans l'os est ainsi tout à fait contrôlée.

Une fois la broche de guidage 1 en position, le patient peut être transporté dans un second lieu qui ne nécessite plus de dispositif d'imagerie, par exemple une salle d'opération. L'opérateur peut être le même opérateur que celui qui a réalisé la fixation de la broche de guidage, ou un autre opérateur, par exemple un chirurgien.

La mise en place de la vis pédiculaire par l'opérateur commence par la palpation de la peau du patient pour trouver l'extrémité sectionnée 5a de la tige de guidage 4 de la broche 1. Une fois cette extrémité 5a trouvée, l'opérateur introduit une gaine externe 18 autour de la broche de guidage 1.

Le chirurgien introduit alors dans la gaine externe 18, autour de la tige de guidage 4 de la broche 1 et selon la flèche F4, le trocart de retrait 12 en direction de la tête 2 de la broche 1.

En référence à la figure 10, le trocart de retrait 12 est alors solidarisé à la broche par vissage dans le sens horaire selon la flèche F5 et coopération entre le filetage 7 sur la tige de guidage 4 ou la tête 2 de la broche 1 et le taraudage 15 sur la face interne du tube creux 13 du trocart de retrait 12.

Le trocart de retrait 12 et la broche de guidage 1 ainsi solidarisés, ces deux pièces sont extraites par préhension manuelle ou motorisée selon la flèche F5 (figure 11). Reste alors uniquement la gaine externe 18 (figure 12) permettant à l'opérateur d'introduire et d'impacter une nouvelle broche ou une vis pédiculaire selon la trajectoire de la broche de guidage.

La figure 13 présente une variante avantageuse de réalisation de l'instrumentation de l'invention dans laquelle la broche 1a est identique à la broche 1 de la figure 4 sauf en ce qu'elle ne comporte pas le filetage 7. En effet, dans cette variante, la partie (ici mâle) du système de solidarisation par baïonnette forme à la fois premier et second élément de couplage. De manière générale, et ce pour toutes les variantes de réalisation de l'invention, les premier et second moyens de couplage sont confondus lorsque le trocart de retrait est identique à la seconde partie du trocart de pose (8, 8b, 8c).

Lorsque la broche présente cette configuration, la partie creuse 9 du trocart de pose 8 (identique à celle des figures 2 et 3) forme alors trocart de retrait 12a pour lequel la tige creuse 13a ne comporte pas de taraudage, la partie 10 (ici femelle) du système de solidarisation par baïonnette 11 permettant d'assurer la solidarisation du trocart de retrait 12a avec la broche 1 en vue de l'extraction de cette broche 1.

Cette configuration présente l'avantage d'une instrumentation complète palliant à tous les inconvénients évoqués précédemment avec uniquement deux pièces : la broche de l'invention, et un trocart (de retrait) ou autrement dénommée partie creuse du trocart de pose amoviblement solidarisé(e) à ladite broche 1.

En variante représentée sur les figures 14 à 16, le système de solidarisation amovible 20 de la partie creuse 9 du trocart de pose 8b avec la broche 1b comporte un filetage 21 situé au niveau de l'extrémité libre 5 de la tige de guidage 4 de la broche 1b qui coopère avec un écrou 22 situé au niveau de la poignée de préhension 9a du trocart de pose 8b via un taraudage (non visible sur les figures) ménagé sur la face interne creuse de l'écrou 22. Ainsi, la désolidarisation de la partie creuse 9 de la broche 1b s'effectue par rotation de l'écrou 22, ce qui évite tout risque, lors de la cette désolidarisation, que la tête 2 de la broche 1b ne tourne sur elle-même lorsque le patient présente une certaine fragilité des os.

Selon une alternative de réalisation de cette variante représentée aux figures 17 à 19, l'écrou taraudé 24 formant partie du trocart de pose 8c, est désolidarisé de la partie creuse dudit trocart de pose 8c, ou tout du moins est-il totalement libre à rotation par rapport à la partie creuse, comme cela sera décrit plus précisément ci-après.

En référence aux figures 17 à 22, un autre mode de réalisation de la broche de guidage 1c et du trocart de pose 8c selon l'invention vont maintenant être décrits.

Comme pour le mode de réalisation du trocart de pose 8 relatif aux figures 2 et 3, le trocart de pose 8c relatif aux figures 17 à 19 comprend la broche de guidage 1c et la partie creuse 13b.

La tête 2b de la broche de guidage 1c comprend à son extrémité libre la pointe 3b destinée à pénétrer dans l'os durant l'implantation. Cette pointe 3b comprend un usinage en double biseau facilitant la pénétration de la tête 2b dans l'os. Alternativement, cette pointe 3b est une pointe diamant. La tête 2b comprend à son extrémité opposée un orifice borgne 32 (voir figures 20 à 22), par exemple de section circulaire ou polygonale, dans lequel la tige de guidage 4c est destinée à être insérée et solidarisée à ladite tête 2b. Dans une variante possible, la tête 2b et la tige 4b sont monobloc. La tête de guidage 2b comprend également une portion 31 présentant en section une forme polygonale, ainsi qu'un filetage 7b formant deuxième moyen de couplage avec le trocart de retrait, qui seront décrits plus loin.

La tige de guidage 4b de la broche 1c est filetée sur une portion éloignée de la tête de la broche. Ce filetage 21b étant destiné à coopérer avec l'écrou taraudé 24 qui est un boulon 24 dans ce mode de réalisation, et forme ainsi première partie du moyen de couplage 6 entre la broche de guidage 1c et la partie creuse 13b du trocart de pose 8c. Préférentiellement, la longueur de la tige de guidage 4b est adaptée de sorte que le filetage 21b soit ménagé au niveau de l'extrémité libre 5b de ladite tige 4b. Néanmoins l'orifice coaxial du boulon 24, dans lequel est ménagé le taraudage 25 destiné à coopérer avec le filetage 21b du premier moyen de couplage 6, peut-être traversant pour permettre la solidarisation dudit boulon 24 à une tige 4b dont la longueur est plus importante que la partie creuse 13b du trocart de pose.

En outre, la portion 31 de la tête de guidage 2b présentant en section un profil de forme polygonale, par exemple hexagonale, est préférentiellement éloignée de la pointe 3b, le reste de la tête 28 présentant en section une forme circulaire. En outre, le diamètre de la portion à section circulaire 28 est supérieur au diamètre de la portion à section polygonale 31 : un épaulement 33 est donc présent au niveau de la jonction entre la portion à section circulaire 28 et la portion à section polygonale 31.

La portion à section polygonale 31 ménagée sur la tête 2b forme un élément de blocage à rotation de ladite tête 2b par rapport à la partie creuse 13b du trocart de pose 8c, lorsque cette partie creuse est engagée sur la broche de guidage 1c. Cet élément de blocage 31 forme la seconde partie des moyens de couplage 6 de la broche de guidage 1c.

L'élément de blocage 31 de la broche de guidage 1c est destiné à coopérer avec une clé 26 ménagée dans la partie creuse 13b du trocart de pose 8c, qui à l'instar du trocart 8 de pose des figures 2 et 3, comprend une tige creuse terminée par des moyens de préhension et d'enfoncement, ces moyens pouvant être une poignée ou un organe d'entrainement 29 destiné à coopérer avec un outil motorisé. Ainsi la clé polygonale 26 du trocart de pose 8c est ménagée au niveau de l'extrémité de la partie creuse 13b dudit trocart 8c opposée aux moyens de préhension et d'enfoncement. La clé 26 est elle-même prolongée par une portion de tube cylindrique 27 dont le diamètre intérieur est supérieur au diamètre intérieur de la clé polygonale 26. Il y a donc un épaulement 34 présent au niveau de la jonction entre la portion de tube 27 et la clé polygonale 26.

Lorsque la partie creuse 13b du trocart de pose 8c est emmanchée à coulissement sur la tige de guidage 4b de la broche 1c, la portion de tube 27 de ladite partie creuse 13b du trocart s'engage autour de la portion à section circulaire 28 de la tête et la clé polygonale 26 s'engage autour de l'élément de blocage 31 à rotation de la tête 2b de la broche 1c, ce qui permet de bloquer toute rotation de la broche 1c par rapport au reste du trocart 8c. En outre, les deux épaulements 33, 34 viennent en appui de contact l'un contre l'autre lorsque la broche 1c et le reste du trocart de pose 8c sont en bonne position pour solidarisation. Le boulon 24 est alors vissé autour du filetage correspondant 21b de la tige de guidage 4b et formant partie du premier moyen de couplage 6 pour solidariser la broche 1c à la partie creuse 13b du trocart de pose pour former le trocart de pose 8c. L'effet combiné du contact entre les épaulements 33, 34, de l'engagement de la clé 26 autour de la portion polygonale 31 de la tête 2b et de la mise en place du boulon 24 autour de la tige 4b bloque totalement en translation et en rotation la broche de guidage 1c par rapport au reste du trocart de pose. Ainsi le système de solidarisation amovible du trocart de pose 8c est formé par le filetage 21b de la tige 4b, la portion hexagonale 31 de la tête 2b, la clé 26 et le taraudage 25 du boulon 24.

En référence aux figures 18 et 19, lorsque les moyens de préhension et d'enfoncement sont formés par l'organe d'entrainement polygonal 29 destiné à coopérer avec un outil motorisé, le boulon 24 est agencé pour s'engager dans un évidement cylindrique 30 ménagé dans la paroi dudit organe d'entrainement 29, de sorte que le boulon 24 soit disposé coaxialement audit organe d'entrainement 29 et donc à la partie creuse 13b du trocart de pose 8c. Ce boulon 24 de forme essentiellement cylindrique peut être complétement désolidarisé de la partie creuse 13b du trocart de pose 8c lorsqu'il n'est pas vissé à la tige 4b. De manière alternative, le boulon 24 peut être monté à rotation dans l'évidement considéré 30, par exemple à l'aide d'une collerette (non représentée) faisant saillie de la paroi cylindrique 35 de l'évidement 30 et coopérant avec une gorge (non représentée) ménagée dans la paroi cylindrique du boulon 24. Cet aménagement particulier évite la perte du boulon 24 et permet de fixer la broche 1c à la partie creuse 13b du trocart de pose 8c sans qu'il n'y ait de rotation entre la broche 1c et la partie creuse 13b du trocart 8c.

Enfin, la broche de guidage 1c comprend un filetage 7b formant le second moyen de couplage, destiné à coopérer avec le trocart de retrait.

Ce filetage 7b peut être ménagé, comme cela a été décrit plus haut, sur la tige 4b de la broche de guidage 1c. Avantageusement, dans le mode de réalisation représenté sur les figures 17 à 22, le filetage 7b est ménagé sur une portion de la tête proche de la tige 4b, et encore préférentiellement sur la portion à section polygonale 31 de sorte que cette section 31 comprenne le filetage 7b et une partie lisse hexagonale 23, comme cela est représenté sur la figure 22 notamment.

De la sorte, le filetage 7b présente une double fonction : celle de former le second moyen de couplage de la broche de guidage 1c au trocart de retrait, et celle d'assurer, avec la partie lisse à section polygonale 23, le blocage à rotation de la broche 1c par rapport au reste du trocart de pose 8c. La réalisation du filetage 7b sur la portion à section polygonale 31 permet donc de profiter de la longueur dudit filetage 7b dans la longueur totale de la portion à section polygonale 31, ce qui assure une mise en prise de la broche 1c avec la partie creuse 13b du trocart de pose 8c particulièrement stable et efficace, avec la génération d'un bras de levier maximal lorsque le praticien exerce un mouvement de rotation sur le trocart de pose 8c pour implanter la tête de guidage 2b dans l'os.

Selon une autre variante représentée sur la figure 16, la partie creuse 9 du trocart de pose 8b peut s'étendre longitudinalement jusqu'à l'extrémité 3 de la tête 2 de la broche en présentant un diamètre supérieur au diamètre D1 de la tête 2 de la broche 1b.

Comme pour la variante illustrée sur la figure 13, il est possible de prévoir que la partie creuse du trocart de pose 8b forme également trocart de retrait, auquel cas le filetage 7 de la broche 1b n'est pas nécessaire. Dans ce cas précis, la tête de guidage 2 ne comprend pas de portion à section polygonale, car le trocart unique ne comprend aucun moyen coopérant susceptible de jouer le rôle de clé.

## Revendications

1. Trocart de pose d'une broche de guidage intra-osseux, comportant une première partie comprenant la broche de guidage (1, 1a, 1b, 1c), ladite broche comportant une tête (2, 2b) destinée à être implantée de façon provisoire dans un os (16), et une tige de guidage (4, 4b) coaxiale et solidaire de la tête (2, 2b), terminée par une extrémité libre (5, 5a, 5b), et adaptée pour au moins affleurer au niveau du plan cutané (17) du patient lorsque la broche (1, 1a, 1b, 1c) est en position implantée dans l'os (16), le trocart de pose (8, 8b, 8c) comprenant en outre une seconde partie creuse (9, 13a, 13b) à laquelle la première partie (1, 1a, 1b, 1c) est couplée de façon amovible au moyen d'un système de solidarisation (11, 20) comportant un premier moyen de couplage (21, 21b, 31) ménagé sur ladite broche (1, 1a, 1b, 1c) et un moyen de couplage coopérant (22; 24) ménagé sur le trocart de pose (8, 8b, 8c), la tige de guidage (4, 4b) de la broche de guidage (1, 1a, 1b, 1c) étant emmanchée à coulissement à l'intérieur de la partie creuse (9, 13a, 13b), et la broche (1, 1a, 1b, 1c) comportant un second moyen de couplage (6, 7, 7b) destiné à assurer au moins sa solidarisation avec un trocart de retrait (8, 12, 12a), **caractérisée en ce que** le premier moyen de couplage (21, 21b) et le moyen de couplage coopérant (22, 24) du système de solidarisation (25) sont situés au niveau de l'extrémité libre de la partie creuse (9, 9a, 29) du trocart de pose (8b, 8c) ; et **en ce que** le premier moyen de couplage comprend en outre un élément de blocage en rotation (31) ménagé au niveau de la tête (2, 2b) de la broche de guidage.

2. Trocart de pose selon la revendication **1, caractérisé en ce que** l'extrémité libre de la partie creuse (9, 13a, 13b) comporte des moyens d'enfoncement (9a, 29) de la tête de guidage (2, 2b) dans l'os, notamment des moyens d'entraînement en rotation (29), et le cas échéant de préhension (9a, 14).

3. Trocart de pose selon la revendication **1** ou **2, caractérisé en ce que** le premier moyen de couplage du système de solidarisation amovible (20) comporte un filetage (21, 21b) réalisé à distance de la tête (2, 2b) de la broche de guidage (1, 1a, 1b, 1c), et **en ce que** le moyen de couplage coopérant comporte un taraudage coïncidant avec ledit filetage (21, 21b).

4. Trocart de pose selon la revendication **3, caractérisé en ce que** le taraudage est réalisé dans un écrou (22, 24) disposé coaxialement à ladite partie creuse (9, 13a, 13b) du trocart de pose (8, 8b, 8c), lequel écrou (22, 24) coopère avec le filetage (21, 21b) pour que la rotation de l'écrou (22, 24) entraîne le vissage ou dévissage de la tige de guidage (4, 4b) de la broche (1, 1a, 1b, 1c) relativement au reste du trocart de pose (8, 8b, 8c) pour permettre la solidarisation ou la désolidarisation de la broche de guidage (1, 1a, 1b, 1c) à la seconde partie creuse (9, 13a, 13b) du trocart de pose (8, 8b, 8c).

5. Trocart de pose selon la revendication **3** ou **4, caractérisé en ce que** le taraudage (25) est situé au niveau des moyens d'enfoncement du trocart de pose (8, 8b, 8c).

6. Trocart de pose selon l'une quelconque des revendications **1** à **5, caractérisé en ce que** le système de solidarisation amovible (11, 20) comporte en outre un élément de blocage à rotation (31) ménagé sur la broche (1c) et un élément de blocage coopérant (26) ménagé dans la partie creuse (13b) du trocart (8c) pour empêcher la rotation de la broche de guidage (1c) par rapport à la partie creuse du trocart de pose selon l'axe dudit trocart de pose.

7. Trocart de pose selon l'une quelconque des revendications **1** à **6, caractérisé en ce que** l'élément de blocage à rotation (31) est situé à proximité de la tige (4b).

8. Trocart de pose selon l'une quelconque des revendications **1** à **7, caractérisé en ce que** l'élément de blocage (31) présente en section une forme polygonale, par exemple hexagonale, et **en ce que** l'élément de blocage coopérant (26) est formé par une clé ménagée dans la partie creuse (13b) du trocart (8c) et adaptée pour coopérer avec le premier élément de blocage (31).

9. Trocart de retrait d'une broche de guidage intra-osseux pour préparer une trajectoire de guidage en vue de l'implantation d'une vis pédiculaire ou orthopédique, la broche comportant une tête (2, 2b) destinée à être implantée de façon provisoire dans un os (16), et une tige de guidage (4, 4b) coaxiale et solidaire de la tête (2, 2b), terminée par une extrémité libre (5, 5a, 5b) adaptée pour au moins affleurer au niveau du plan cutané (17) du patient lorsque la broche (1, 1a, 1b, 1c) est en position implantée dans l'os (16), laquelle broche (1, 1a, 1b, 1c) comporte un premier moyen de couplage (21,21b, 31) destiné à assurer sa solidarisation et désolidarisation avec au moins une partie d'un trocart de pose (8, 8b, 8c); et un second moyen de couplage (6, 7, 7b, 21, 21b) destiné à assurer au moins sa solidarisation avec un trocart de retrait, **caractérisé en ce qu'**il comporte une tige creuse d'attache (13, 13a) destinée à s'emmancher autour de la tige de guidage (4, 4b) de la dite broche (1, 1a, 1b, 1c), et un moyen de couplage (10, 15) coopérant avec le second moyen de couplage (6, 7, 7b) de la dite broche (1, 1a, 1b, 1c) pour former un système de solidarisation amovible permettant le retrait de la broche de guidage (1, 1a, 1b, 1c) lorsque le second moyen de couplage (6, 7, 7b) de la broche (1, 1a, 1b, 1c) et le moyen de couplage coopérant (10, 15, 22) de la tige creuse (13, 13a) du trocart de retrait (8, 8b, 12, 12a) sont assemblés, et **en ce que** le premier moyen de couplage comprenant en outre un élément de blocage en rotation (31) ménagé au niveau de la tête (2, 2b) de la broche de guidage.

10. Trocart de retrait selon la revendication **9, caractérisé en ce que** le moyen de couplage coopérant est constitué d'un taraudage (15) réalisé sur la face interne de la tige creuse (13), et qui est apte à être assemblé au second moyen de couplage (7, 7b) de la broche de guidage (1, 1a, 1b, 1c) constitué par un filetage (7, 7b) réalisé sur la tige de guidage (4) ou sur la partie d'extrémité de la tête (2, 2b) solidaire de la tige de guidage (4b).

## Patentansprüche

1. Positionierungstrokar eines enossalen Führungsstifts, aufweisend einen ersten Teil, der den Führungsstift (1, 1a, 1b, 1c) umfasst, wobei der Stift einen Kopf (2, 2b) aufweist, der bestimmt ist, vorübergehend in einen Knochen (16) implantiert zu sein, und eine koaxiale, mit dem Kopf (2, 2b) fest verbundene Führungsstange (4, 4b), die in einem freien Ende (5, 5a, 5b) endet und geeignet ist, um mindestens im Bereich der Hautebene (17) des Patienten bündig zu sein, wenn der Stift (1, 1a, 1b, 1c) in im Knochen (16) implantierter Position ist, wobei der Positionierungstrokar (8, 8b, 8c) ferner einen zweiten hohlen Teil (9, 13a, 13b) umfasst, an den der erste Teil (1, 1a, 1b, 1c) mittels eines Verbindungssystems (11, 20) lösbar gekoppelt ist, aufweisend ein erstes Kopplungsmittel (21, 21b, 31), das auf dem Stift (1, 1a, 1b, 1c) eingerichtet ist, und ein mitwirkendes Kopplungsmittel (22; 24), das auf dem Positionierungstrokar (8, 8b, 8c) eingerichtet ist, wobei die Führungsstange (4, 4b) des Führungsstifts (1, 1a, 1b, 1c) in das Innere des hohlen Teils (9, 13a, 13b) gleitend eingeschoben ist, und der Stift (1, 1a, 1b, 1c) ein zweites Kopplungsmittel (6, 7, 7b) aufweist, das zur Sicherung mindestens seiner festen Verbindung mit einem Rückzugstrokar (8, 12, 12a) bestimmt ist, **dadurch gekennzeichnet, dass** sich das erste Kopplungsmittel (21, 21b) und das mitwirkende Kopplungsmittel (22, 24) des Verbindungssystems (25) im Bereich des freien Endes des hohlen Teils (9, 9a, 29) des Positionierungstrokars (8b, 8c) befinden; und dass das erste Kopplungsmittel ferner ein Rotationsblockademittel (31) umfasst, das im Bereich des Kopfes (2, 2b) des Führungsstifts eingerichtet ist.

2. Positionierungstrokar nach Anspruch **1, dadurch gekennzeichnet, dass** das freie Ende des hohlen Teils (9, 13a, 13b) Eintreibmittel (9a, 29) des Führungskopfes (2, 2b) in den Knochen aufweist, insbesondere Rotationsantriebsmittel (29), und gegebenenfalls Greifmittel (9a, 14).

3. Positionierungstrokar nach Anspruch **1** oder **2, dadurch gekennzeichnet, dass** das erste Kopplungsmittel des lösbaren Verbindungssystems (20) ein Außengewinde (21, 21b) aufweist, das vom Kopf (2, 2b) des Führungsstifts (1, 1a, 1b, 1c) beabstandet ist, und dass das mitwirkende Kopplungsmittel ein Innengewinde aufweist, das mit dem Außengewinde (21, 21b) übereinstimmt.

4. Positionierungstrokar nach Anspruch **3, dadurch gekennzeichnet, dass** das Innengewinde in einer Mutter (22, 24) ausgeführt ist, die koaxial zu dem hohlen Teil (9, 13a, 13b) des Positionierungstrokars (8, 8b, 8c) angeordnet ist, wobei die Mutter (22, 24) mit dem Außengewinde (21, 21b) zusammenwirkt, damit die Rotation der Mutter (22, 24) das Verschrauben oder Entschrauben der Führungsstange (4, 4b) des Stifts (1, 1a, 1b, 1c) relativ zum Rest des Positionierungstrokars (8, 8b, 8c) bewirkt, um das feste Verbinden oder das Lösen des Führungsstifts (1, 1a, 1b, 1c) am bzw. vom zweiten hohlen Teil (9, 13a, 13b) des Positionierungstrokars (8, 8b, 8c) zu gestatten.

5. Positionierungstrokar nach Anspruch **3** oder **4, dadurch gekennzeichnet, dass** sich das Innengewinde (25) im Bereich der Eintreibmittel des Positionierungstrokars (8, 8b, 8c) befindet.

6. Positionierungstrokar nach einem der Ansprüche **1** bis **5, dadurch gekennzeichnet, dass** das lösbare Verbindungssystem (11, 20) ferner ein Rotationsblockadeelement (31) aufweist, das auf dem Stift (1c) eingerichtet ist, und ein mitwirkendes Blockadeelement (26), das in dem hohlen Teil (13b) des Trokars (8c) eingerichtet ist, um die Rotation des Führungsstifts (1c) in Bezug auf den hohlen Teil des Positionierungstrokars gemäß der Achse des Positionierungstrokars zu verhindern.

7. Positionierungstrokar nach einem der Ansprüche **1** bis **6, dadurch gekennzeichnet, dass** sich das Rotationsblockadeelement (31) in der Nähe der Stange (4b) befindet.

8. Positionierungstrokar nach einem der Ansprüche **1** bis 7, **dadurch gekennzeichnet, dass** das Blockadeelement (31) im Querschnitt eine polygonale, beispielsweise sechseckige, Form aufweist, und dass das mitwirkende Blockadeelement (26) von einem Schlüssel gebildet ist, der in dem hohlen Teil (13b) des Trokars (8c) eingerichtet und geeignet ist, mit dem ersten Blockadeelement (31) zusammenzuwirken.

9. Rückzugstrokar eines enossalen Führungsstifts zur Vorbereitung eines Führungswegs zwecks Implantation einer Pedikel- oder orthopädischen Schraube, wobei der Stift einen Kopf (2, 2b) aufweist, der bestimmt ist, vorübergehend in einen Knochen (16) implantiert zu sein, und eine koaxiale, mit dem Kopf (2, 2b) fest verbundene Führungsstange (4, 4b), die in einem freien Ende (5, 5a, 5b) endet und geeignet ist, um mindestens im Bereich der Hautebene (17) des Patienten bündig zu sein, wenn der Stift (1, 1a, 1b, 1c) in im Knochen (16) implantierter Position ist, wobei der Stift (1, 1a, 1b, 1c) ein erstes Kopplungsmittel (21, 21b, 31) aufweist, das bestimmt ist, sein festes Verbinden und das Lösen mit bzw. von mindestens einem Teil des Positionierungstrokars (8, 8b, 8c) zu sichern, und ein zweites Kopplungsmittel (6, 7, 7b, 21, 21b), das zum Sichern mindestens seiner festen Verbindung mit einem Rückzugstrokar bestimmt ist, **dadurch gekennzeichnet, dass** er eine hohle Verbindungsstange (13, 13a) aufweist, die bestimmt ist, sich um die Führungsstange (4, 4b) des Stifts (1, 1a, 1b, 1c) zu schieben, und ein Kopplungsmittel (10, 15), das mit dem zweiten Kopplungsmittel (6, 7, 7b) des Stifts (1, 1a, 1b, 1c) zusammenwirkt, um ein lösbares Verbindungssystem zu bilden, das den Rückzug des Führungsstifts (1, 1a, 1b, 1c) gestattet, wenn das zweite Kopplungsmittel (6, 7, 7b) des Stifts (1, 1a, 1b, 1c) und das mitwirkende Kopplungsmittel (10, 15, 22) der hohlen Stange (13, 13a) des Rückzugstrokars (8, 8b, 12, 12a) verbunden sind, und dass das erste Kopplungsmittel ferner ein Rotationsblockadeelement (31) umfasst, das im Bereich des Kopfes (2, 2b) des Führungsstifts eingerichtet ist.

10. Rückzugstrokar nach Anspruch **9, dadurch gekennzeichnet, dass** das mitwirkende Kopplungsmittel aus einem Innengewinde (15) besteht, welches auf der Innenseite der hohlen Stange (13) ausgeführt ist und das imstande ist, mit dem zweiten Kopplungsmittel (7, 7b) des Führungsstifts (1, 1a, 1b, 1c) verbunden zu sein, das von einem Außengewinde (7, 7b) gebildet ist, das auf der Führungsstange (4) oder auf dem Endteil des Kopfes (2, 2b) ausgeführt ist, der mit der Führungsstange (4b) fest verbunden ist.

## Claims

1. Trocar for positioning an endosteal guide pin, comprising a first part comprising the guide pin (1, 1a, 1b, 1c), said pin including a head (2, 2b) intended to be provisionally implanted in a bone (16), and a guide rod (4, 4b), coaxial with and rigidly connected to the head (2, 2b), ending in a free end (5, 5a, 5b), and adapted such that it is at least flush with the skin surface (17) of the patient when the pin (1, 1a, 1b, 1c) is in the implanted position in the bone (16), the positioning trocar (8, 8b, 8c) further comprising a second hollow part (9, 13a, 13b) to which the first part (1, 1a, 1b, 1c) is coupled in a removable manner by means of a rigid connection system (11, 20) including a first coupling means (21, 21b, 31) provided on said pin (1, 1a, 1b, 1c) and a cooperating coupling means (22; 24) provided on the positioning trocar (8, 8b, 8c), the guide rod (4, 4b) of the guide pin (1, 1a, 1b, 1c) being fitted inside the hollow part (9, 13a, 13b) such that it slides, and the pin (1, 1a, 1b, 1c) comprising a second coupling means (6, 7, 7b,) intended at least to ensure the rigid connection thereof to a removal trocar (8, 12, 12a), **characterised in that** the first coupling means (21, 21b) and the cooperating coupling means (22, 24) of the bonding system (25) are located at the free end of the hollow part (9, 9a, 29) of the positioning trocar (8, 8b, 8c); and said first coupling means further comprises a rotation locking element (31) produced at the head (2, 2b) of the guide pin.

2. Positioning trocar according to the claim **1, characterised in that** the free end of the hollow part (9, 13a, 13b) includes driving means (9a, 29) for driving the guide head (2, 2b) into the bone, in particular rotating means (29) and, where appropriate, grasping means (9a, 14).

3. Positioning trocar according to claim **1** or **2, characterised in that** the first coupling means of the removable rigid connection system (20) includes a threading (21, 21b) made away from the head (2, 2b) of the guide pin (1, 1a, 1b, 1c), and **in that** the cooperating coupling means includes a tapping coincident with said threading (21, 21b).

4. Positioning trocar according to the claim **3, characterised in that** the tapping is produced in a nut (22, 24) disposed coaxially to said hollow part (9, 13a, 13b) of the positioning trocar (8, 8b, 8c), which nut (22, 24) cooperates with the threading (21, 21b) such that the rotation of the nut (22, 24) drives the screwing or unscrewing of the guide rod (4, 4b) of the pin (1, 1a, 1b, 1c) relative to the rest of the positioning trocar (8, 8b, 8c) in order to enable the guide pin (1, 1a, 1b, 1c) to be rigidly connected to or disconnected from the second hollow part (9, 13a, 13b) of the positioning trocar (8, 8b, 8c).

5. Positioning trocar according to claim **3** or claim **4, characterised in that** the tapping (25) is located at the driving means of the positioning trocar (8, 8b, 8c).

6. Positioning trocar according to any one of claims **1** to **5, characterised in that** the removable rigid connection system (11, 20) further includes a rotation locking element (31) provided on the pin (1c) and a cooperating locking element (26) provided in the hollow part (13b) of the trocar (8c) in order to prevent the rotation of the guide pin (1c) relative to the hollow part of the positioning trocar around the axis of said positioning trocar.

7. Positioning trocar according to the any one of claims **1** to **6, characterised in that** the rotation locking element (31) is located in the vicinity of the rod (4b).

8. Positioning trocar according to any one of claims **1** to 7, **characterised in that** the locking element (31) has a polygon-shaped section, for example a hexagonal section, and **in that** the cooperating locking element (26) is formed by a key made in the hollow part (13b) of the positioning trocar (8c) and adapted for cooperating with the first locking element (31).

9. Trocar for removing an endosteal guide pin for preparing a guide path for the implantation of a pedicle or orthopaedic screw, the pin including a head (2, 2b) intended to be provisionally implanted in a bone (16), and a guide rod (4, 4b), coaxial with and rigidly connected to the head (2, 2b), ending in a free end (5, 5a, 5b) adapted such that it is at least flush with the skin surface (17) of the patient when the pin (1, 1a, 1b, 1c) is in the implanted position in the bone (16), which pin (1, 1a, 1b, 1c) includes a first coupling means (21, 21b, 31) intended to ensure the rigid connection thereof to and the disconnection thereof from at least one part of a positioning trocar (8, 8b, 8c), and a second coupling means (6, 7, 7b, 21, 21b) intended at least to ensure the rigid connection thereof to a removal trocar, **characterised in that** it includes a hollow fixing rod (13, 13a) intended to be fitted around the guide rod (4, 4b) of said pin (1, 1a, 1b, 1c), and a coupling means (10, 15) cooperating with the second coupling means (6, 7, 7b) of said pin (1, 1a, 1b, 1c) to form a removable rigid connection system enabling the guide pin (1, 1a, 1b, 1c) to be removed when the second coupling means (6, 7, 7b) of the pin (1, 1a, 1b, 1c) and the cooperating coupling means (10, 15, 22) of the hollow rod (13, 13a) of the removal trocar (8, 8b, 12, 12a) are assembled together; and said first coupling means further comprises a rotation locking element (31) produced at the head (2, 2b) of the guide pin.

10. Removal trocar according to claim **9, characterised in that** the cooperating coupling means is formed by a tapping (15) made on the inside face of the hollow rod (13), and which is capable of being assembled with the second coupling means (7, 7b) of the guide pin (1, 1a, 1b, 1c) formed by a threading (7, 7b) made on the guide rod (4) or on the end part of the head (2, 2b) rigidly connected to the guide rod (4b).
